# EUROPEAN PATENT APPLICATION

(11) **EP 2 204 651 A1**
(43) Date of publication of application: **07.07.2010**
(21) Application number: 09180837.8
(22) Date of filing: 29.12.2009
(51) Int. Cl.: G01N 33/543

(54) **Electrosensing antibody-probe detection and measurement sensor and method**

(30) Priority: 06.01.2009 US 142687 P
(71) Applicant: Shiming, Lin, Taipei (TW)
(72) Inventor: Lin, Shiming, Taipei (TW); Lee, Shih-yuan, Taipei (TW); Bor-Ching, Sheu, Taipei (TW); Lin, Chih-chen, Kaohsiung County (TW); Lin, Pan-chien, Hsinchu County (TW)
(74) Representative: Becker Kurig Straus

(57) **Abstract**

A sensor (400) and its method for electrosensing an antigen (432) in a test sample are disclosed. The sensor (400) has two electrodes (412, 414) electrically disconnected and physically separated from each other, and a layer of antibody (422) is immobilized on the surface of at least one of the electrodes (412, 414). The antibody (422) has specific binding reactivity with the antigen (432). Conductivity promotion molecules (442) may be tethered over and/or distributed between the antibody-populated electrodes (412, 414) for improving electrical conductivity characteristics across the two electrodes. The antibody (422) captures the antigen (432) present in the test sample mixed in a buffer solution that comes into contact with the antibody-populated electrodes. This alters the electrical conductivity characteristic across the two electrodes (412, 414) in which an amount representative of the altering provides an indication for electrosensing of the antigen (432).

## Description

### BACKGROUND

### Field of the Invention

The present invention relates in general to electrosensing detection and measurement using antibody as probe. In particular, the present invention relates to an electrosensing sensor with antibody probe and its related method.

### Description of Related Art

Detection of the presence of target substance in test sample using biochip in medical and related applications is known. Based on factors such as precision and cost, biochip sensors are used for the detection of presence of their designed targets. If possible in terms of available technology and allowable in costs, sensing beyond the mere detection of presence of the target substance is obviously more useful in every application imaginable. For example, in biomedical applications, an indication of the level of presence of a target substance, for example, concentration in a scale of from 1 to 10, 1 to 100 or even higher resolution and with accuracy, would be very informative for the intended purpose of such sensing.

Biochips based on optical sensing are among the most common nowadays. These chips rely on optical sensory that requires bulky and costly precision instruments for the reading of the result of sensing reaction on the chip. To circumvent these problems, biochips based on electrosensing appear to be reasonable. Examination (or, sensing) of an electrosensing biochip after exposure to test sample is electric. The information sensed from a test sample is an electrical parameter that can be the value of resistance, conductance, current, or any other that is useful.

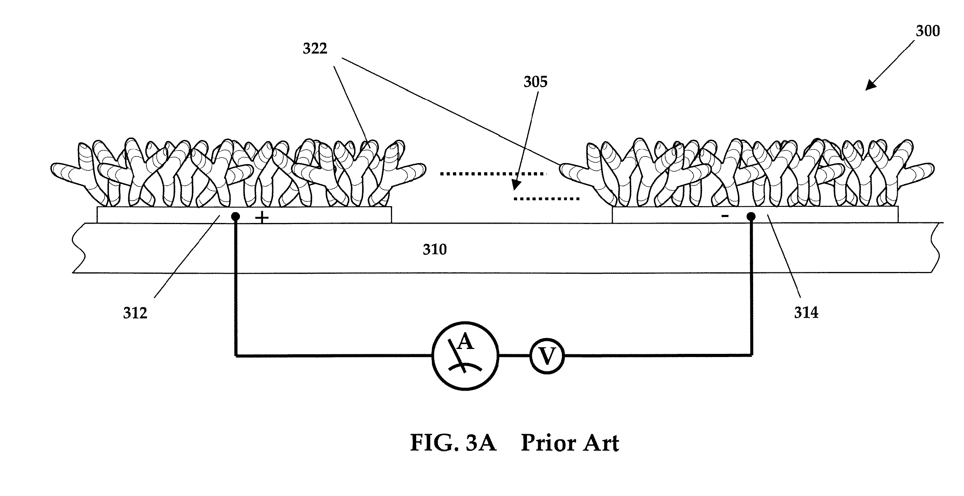

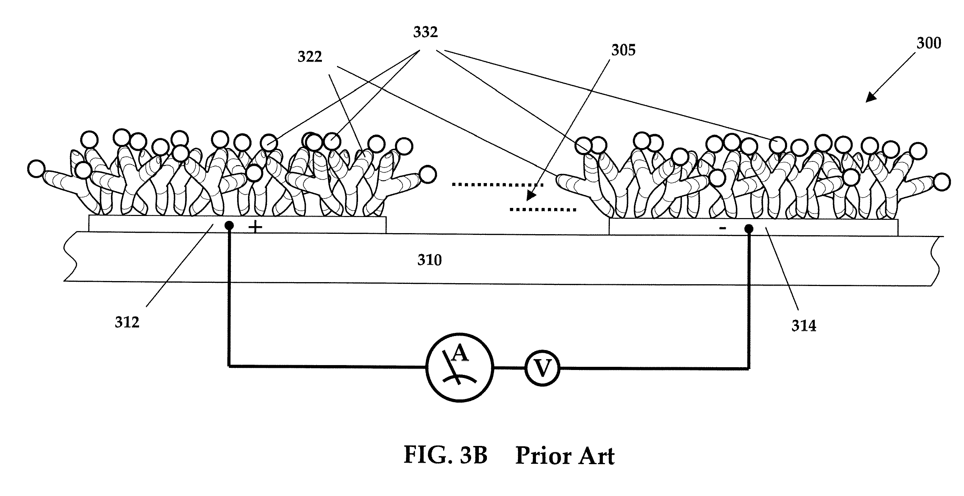

However, electrosensing technology has so far been limited in use due to the fact that most fluidic test samples are inherently electrically non-conductive. FIGS. 3A and 3B illustrate how they are not ideal for the sensing of general antigen targets using antibody as the probe. For example, FIG. 3A schematically illustrates a prior art sensor chip 300 having antibody molecules 322 such as immunoglobulin G immobilized onto the surface of its positive 312 and negative 314 electrodes, which are, for example, thin films of Au, Ag, Cu or Ni etc. To be useful, this system must allow for detectable changes in electrical current in the environment generally indicated by reference numeral 305 between the electrodes of the sensor chip.

Electrical conductivity between the electrodes of the sensor system after a test sample is introduced, however, is substantially poor, such as is schematically depicted in FIG. 3B, when antigen molecules 332 - most of which non- or poorly conductive in nature - in the sample are bound to the antibody molecules 322 populated on the electrode surfaces. Conventional electro-sensing biochips are thus only applicable to testing in which enzyme or catalyst is used as probe on the chip. Applications are therefore limited.

### SUMMARY OF THE INVENTION

It is therefore an object of the present invention to provide an electrosensing antibody-probe chip and its related method for the sensing of presence of various target substances.

It is also an object of the present invention to provide an electrosensing antibody-probe chip and its related method for the sensing measurement of the level of presence of various target substances.

It is another object of the present invention to provide an electrosensing antibody-probe chip and its related method for the detection and measurement of target substances that is easy, small and low-cost to implement because no bulky, high-precision and therefore costly hardware is required.

It is yet another object of the present invention to provide an electrosensing antibody-probe chip and its related method that is suitable for the testing of vastly expanded target substances for applications beyond biomedical such as environmental control and industrial.

The present invention achieves the above and other objects by promoting electrical conductivity in the sensor chip system (the chip and the test fluidic sample it reacts). In a sense, the antibody probe molecules of the sensor chip and method of the present invention literally "wears an electrically conductive tights" so that the electrical conductivity in the system becomes "amplified" to a level sensible by today's instrumentation. Measured electrical parameter such as resistance of the sensor chip system thus becomes a detectable and discernable and therefore meaningful parameter for interpretation.

In one embodiment the present invention achieves the above and other objects by providing a sensor for electrosensing an antigen in a test sample that comprises two electrodes electrically disconnected and physically separated from each other and a layer of antibody immobilized on the surface of at least one of the electrodes, the antibody having specific binding reactivity with the antigen. The antibody captures the antigen present in the test sample mixed in a buffer solution that comes into contact with the antibody-populated electrodes thereby altering electrical conductivity characteristic across the two electrodes whereby an amount representative of the altering providing an indication for electrosensing of the antigen.

In another embodiment the present invention achieves the above and other objects by providing a sensor for electrosensing an antigen in a test sample that comprises two electrodes electrically disconnected and physically separated from each other; a layer of antibody immobilized on the surface of at least one of the electrodes, the antibody having specific binding reactivity with the antigen; and conductivity promotion molecules tethered over and/or distributed between the antibody-populated electrodes for improving electrical conductivity characteristics across the two electrodes. The antibody captures the antigen present in the test sample mixed in a buffer solution that comes into contact with the antibody-populated electrodes thereby altering the improved electrical conductivity characteristic across the two electrodes whereby an amount representative of the altering providing an indication for electrosensing of the antigen.

In another embodiment the present invention achieves the above and other objects by providing a method of electrosensing an antigen in a test sample using a sensor, where the sensor has two electrodes electrically disconnected and physically separated from each other and a layer of antibody immobilized on the surface of at least one of the electrodes, and he antibody has specific binding reactivity with the antigen. The method comprises measuring electrically across the electrodes after the test sample comes into contact with the antibody-populated electrodes. The antibody captures the antigen present in the test sample thereby altering electrical conductivity characteristic across the two electrodes whereby an amount representative of the altering providing an indication for electrosensing of the antigen.

In another embodiment the present invention achieves the above and other objects by providing a method of electrosensing an antigen in a test sample using a sensor, where the sensor has two electrodes electrically disconnected and physically separated from each other and a layer of antibody immobilized on the surface of at least one of the electrodes, and the antibody has specific binding reactivity with the antigen. The method comprises tethering conductivity promotion molecules over and/or distributing between the antibody-populated electrodes for improving electrical conductivity characteristics across the two electrodes; and measuring electrically across the electrodes after the test sample comes into contact with the antibody-populated electrodes. The antibody captures the antigen present in the test sample thereby altering the improved electrical conductivity characteristic across the two electrodes whereby an amount representative of the altering providing an indication for electrosensing of the antigen.

### BRIEF DESCRIPTION OF THE DRAWINGS

FIG. 1 illustrates the outline of a basic electrosensing system.
FIGS. 2A and 2B show two of the possible configurations of the sensor chip.
FIGS. 3A and 3B explains how conventional electrosensing is not suitable for testing antigens using antibody probes.
FIGS. 4A-4C respectively shows the preparation of an embodiment of the sensor chip of the present invention and its testing and sensing of a sample.
FIGS. 5A-5C respectively shows the preparation of another embodiment of the sensor chip of the present invention and its testing and sensing of a sample.
FIG. 6 schematically describes how the electrosensing chip and method of the present invention is practically useful.
FIGS. 7A and 7B illustrate two examples of the process for surface modification of sensor chips of the present invention that promotes electrical conductivity in the test system.
FIGS. 8-10 respectively show results of various sample testing using sensors of the present invention.
FIG. 11 illustrates the outline of an embodiment of the sensor chip of the present invention.
FIG. 12 outlines the flow channel configuration of a sensor chip processing machine in accordance with an embodiment of the present invention.

### DETAILED DESCRIPTION OF THE PREFERRED EMBODIMENTS

The present invention achieves practical and useful electrosensing by promoting electrical conductivity in the sensor chip system (the chip and the test fluidic sample it reacts with). In a sense, the antibody probe molecules of the sensor chip and method of the present invention literally "wears an electrically conductive tights" so that the electrical conductivity in the system becomes "amplified" to a level sensible by today's instrumentation. Measured electrical parameter such as resistance of the sensor chip system thus becomes a detectable and discernable and therefore meaningful parameter for interpretation.

According to the present invention, the antibodies immobilized on the sensor chip and used as test probes are effectively turned from non-conductors into semi-conducting or even conducting substances. This allows the electrical impedance of an examined sample fluid (after reacting with the antibody on the sensor chip) to become not only detectable but also discernable in terms of precise value by the instrumentation. Such measured reading can be used to interpret the result of the intended sensing.

In fact, as is understandable, other than impedance, electrical parameters such as capacitance of the system all become measurable as a result of the idea of the inventive promotion of electrical conductivity in the system. Also, instead of the strict definition of the reciprocal of electrical resistance, the term "conductivity" as used herein refers to the more general characteristics of the state of electrical conduction. Thus, "conductivity promotion" means "the improvement of the general state of electrical conduction."

Thus, the sensor and method of the present invention are able to establish an electrically conductive environment that allows for any alteration of electrical conductance caused by the presence of captured substance in the environment to become detectable and discernable. Because the sensor and method of the present invention effectively "amplifies" the range of detection of electrical characteristics of the entire test sample system, any alteration of electrical characteristics, electrical impedance or current, or electrical capacitance, measured under either a DC voltage or an AC of selected frequency, is easily detectable and scalable with precision. The amount of such alteration becomes an indication of the level of presence of the target substance in the test sample.

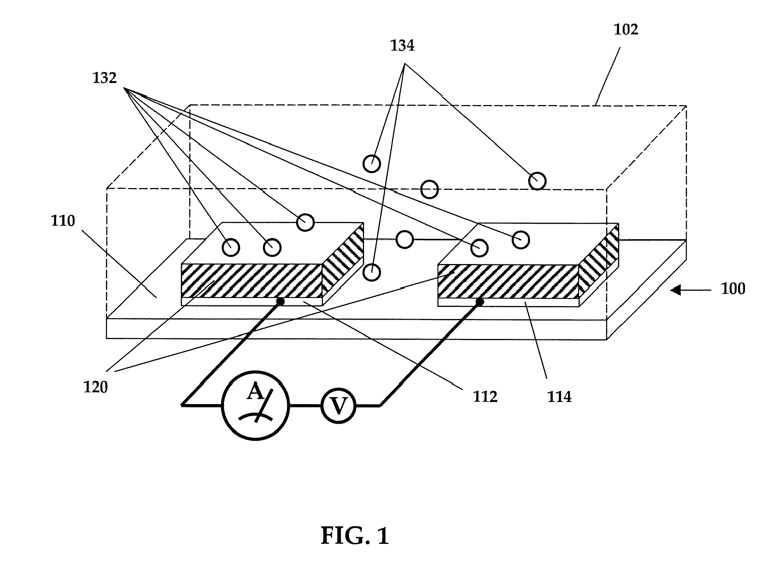

FIG. 1 illustrates the outline of a basic electrosensing system. The sensor chip 100 built on a substrate 110 has layers of antibody probes 120 immobilized onto the surface of its positive and negative electrodes 112 and 114, which may, for example, be thin films of Au, Ag, Cu or Ni etc. Electrodes 112 and 114 serve as the physical base to hold the antibody probes aimed at specific sensing functionality.

An embodiment of the system implementing the inventive electrosensing technique of the present invention is based on a sensor chip 100 that can be incorporated into a test instrument to provide a sensing cavity 102. Inside the cavity, a test sample comes into contact with the chip, allowing target antigen molecules 134 suspending in the fluidic sample to become captured antigen 132 bound to the antibody probe 120.

As will be described in more detail, the system of FIG. 1 allows for a precision measurement of the concentration of target antigen in the test sample. This is via the use of an electric current measurement instrument when an electric voltage is applied across the electrodes of the sensor chip, as is depicted schematically in the drawing.

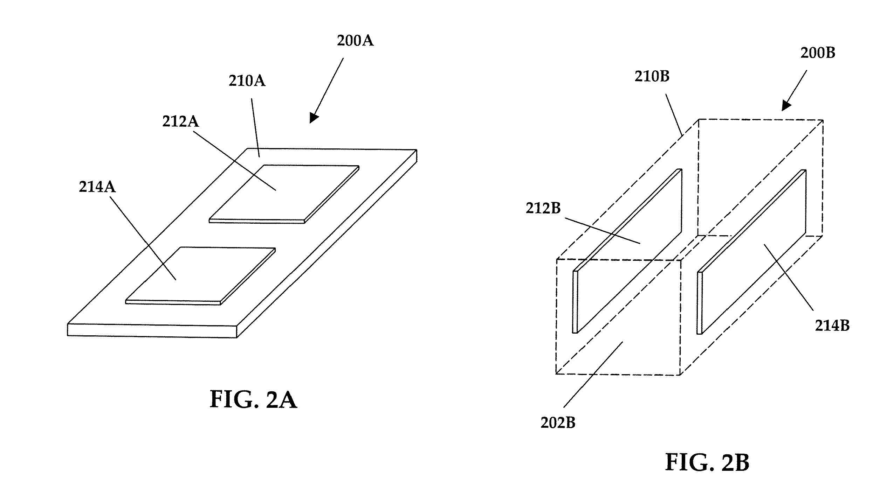

FIGS. 2A and 2B show two of the possible configurations of the sensor chip in accordance with a preferred embodiment of the present invention. The sensor chip 200A of FIG. 2A takes the form of the typical flat chip with sensor electrodes 212A and 214A placed side-by-side on its substrate 210A. Such a flat chip configuration relies on a chip reader apparatus to form a sample cavity in which the sensing may take place.

By contrast, the sensor chip 200B of FIG. 2B is a tubular chip, with its two sensors 212B and 214B attached to the inner surface of the tubular "substrate" 210B at locations generally oppositely facing each other. With such a tubular configuration, the sensor chip 200B is able to easily provide a sample cavity 202B when its both ends are sealed as it is inserted into a corresponding reader apparatus.

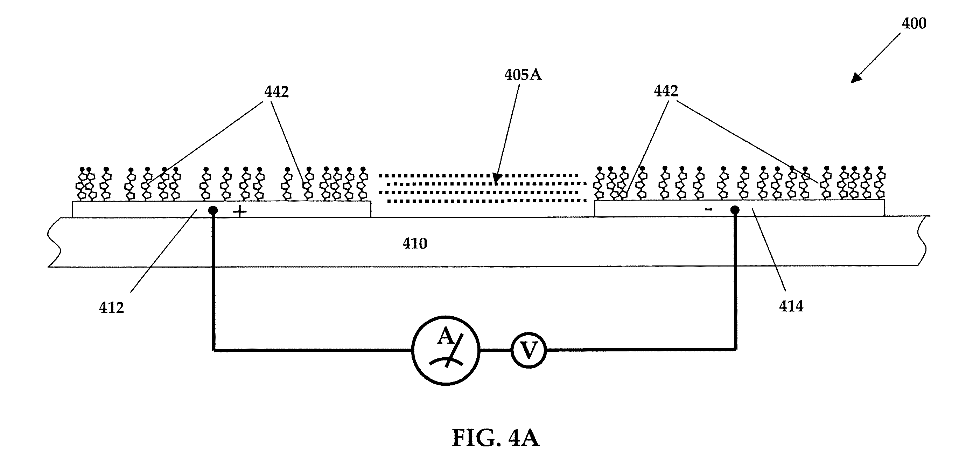

FIGS. 4A-4C respectively shows the preparation of an embodiment of the sensor chip of the present invention and its testing and sensing of a sample. Note that in the drawing, dimensions of the electrodes, the antibody, the antigen and the conductivity promotional molecules are not drawn to scale. Rather, they are illustrated disproportionately and in a manner exaggerated for the purpose explanation of the idea of the present invention.

FIG. 4A shows the basic system of a sensor chip in the electrically conductive environment has its electrical conductivity increased by surface modification using conductivity promotion molecules. In a preferred embodiment, gold is used in the form of thin film to form the basic positive and negative electrodes 412 and 414 for the sensor chip 400 constructed on a substrate 410. Other metals such as Ag, Cu, Ni, etc. can also be used. Depending on application, suitable alloys (ex, indium tin oxide, ITO) can also be used.

Electrically conductive molecules are bound to the electrodes, as is schematically illustrated in the drawing by their immobilization to the surface of electrodes shown by reference numeral 442. These become conductivity promotion molecules immobilized to the surface of the electrodes. This allows the basic sensor system to provide an enhanced electrically conductive environment when the chip is used since conductivity promotion molecules modify the surface characteristics of the sensor chip, which results into the promotion of electrical conductivity of the bare sensor system. Electrical conductivity between the positive and negative electrodes becomes greatly improved for sample testing (that is, after antibody probe molecules are present). This is a system that allows sensible electric current between the electrodes 412 and 414 of the sensor chip 400 because of the much-improved electrically conductive environment generally indicated by reference numeral 405A between the electrodes.

Substances suitable for use as electrical conductivity promotion material include, but is not limited to, oligothiophene-silane, oligothiophene-thiol, (1-phenyl)-oligothiophene, (2-phenyl)-oligothiophene, side-arm oligothiophene, oligophenyl oligothiophene, and the derivatives thereof etc.

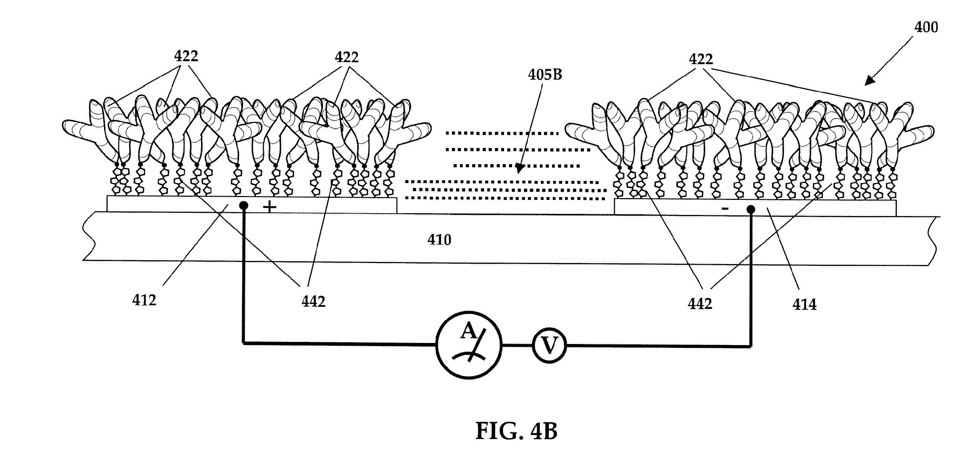

In FIG. 4B, antibody 422 for the intended target-probing application is added to the sensor chip 400 by conjugation with the layer of conductivity promotion molecules 442. With the immobilization of this antibody, conductivity of the sensor chip at this stage (when target antigens are not yet present) in the electrically conductive environment 405B decreases somewhat, but is still well within range for easy instrument gauging.

With the presence of the antibody 422, the chip 400 of FIG. 4B is a ready sensor for its designed target electrosensing application. For any intended sensing application, specific non-conductive antibody molecules are immobilized to the chip. For example, immunoglobulin G molecules can be used as the antibody probe for the testing of antigens such as S100, alpha-fetoprotein, and tropolin I, etc. System conductivity decreased to an extent reflected by the presence of the probe. This change in conductivity becomes a reference value for test measurements.

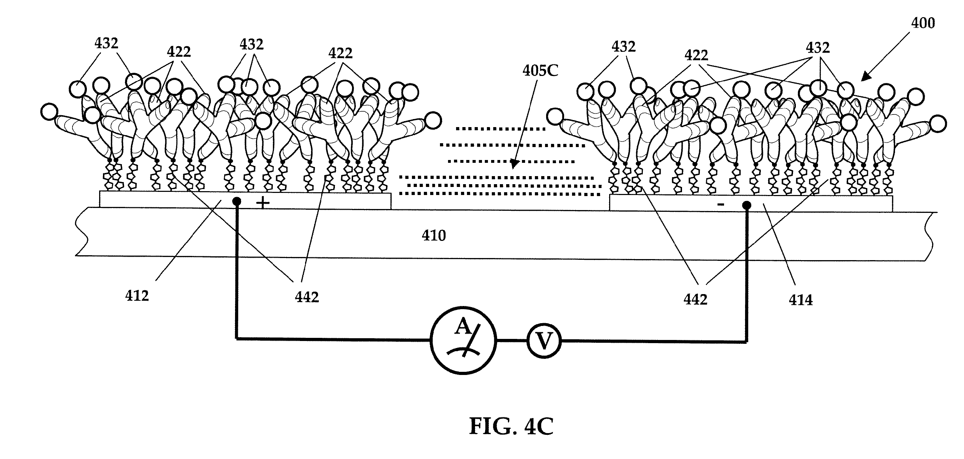

FIG. 4C illustrates the electrosensing of target antigen by exposure to the probe antibody immobilized to the chip. The ready sensor chip 400 of FIG. 4B is exposed to a test sample. With the antibody 422 immobilized as the probe aiming for the binding of specific target, antigen 432, the target present in the sample, is captured by, or, reacts with antibody.

With the presence of captured antigen molecules 432, overall conductivity of the entire electrically conductive environment 405C further changes (compared with FIG. 4B), and the discrepancy of this impedance reading (picked up as the current between the electrodes) is an indication of the level of presence of antigen in the system.

For electrosensing in accordance with the present invention, as a sample containing non-conductive antigen target is introduced into the fluidic detection and measurement environment provided by the sensor chip of FIG. 4C, system conductivity decreases as a result. Such decrease is reflected by corresponding decrease in the measured current. The decrease is at an extent proportionally signifying the level of presence of the target substance as captured by the chip. It is, however, noticeable that in some cases the binding of certain target antigen in the test sample to the antibody probe of the sensor chip does inflict a conductivity increase than when they are not present in the system.

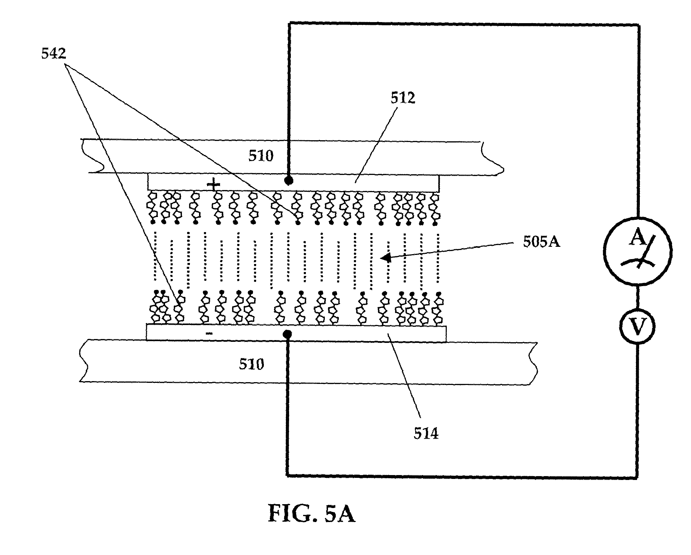

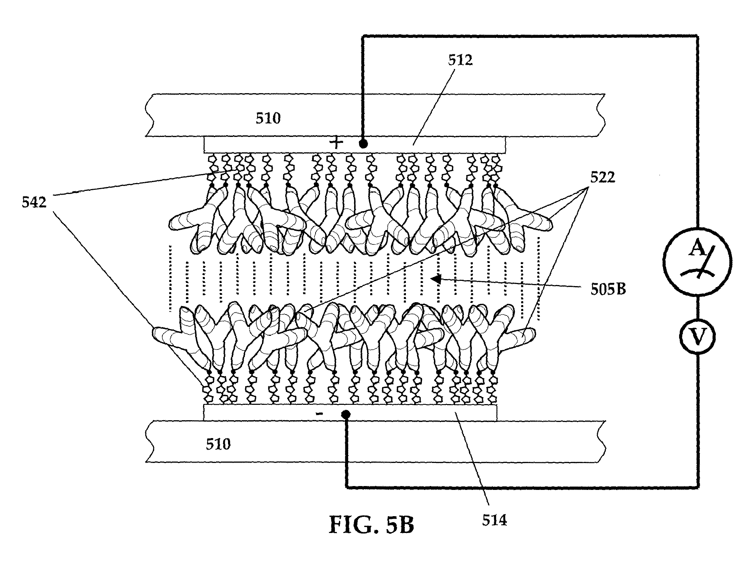

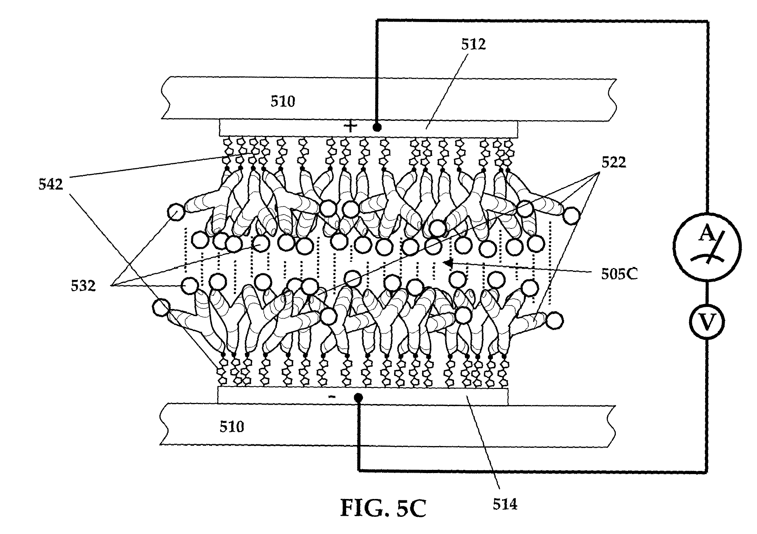

FIGS. 5A-5C respectively shows the preparation of another embodiment of the sensor chip of the present invention and its testing and sensing of a sample. The example described in FIGS. 5A-5C is substantially the same as that of FIGS. 4A-4C except that the physical configuration of the sensor chip has its electrodes arranged in an oppositely facing position. It is theorized, but without limitation thereto, that such opposite-facing configuration for electrodes may allow for improved electrosensing due to improved conductivity conditions then in the flat configuration of FIGS. 4A-4C.

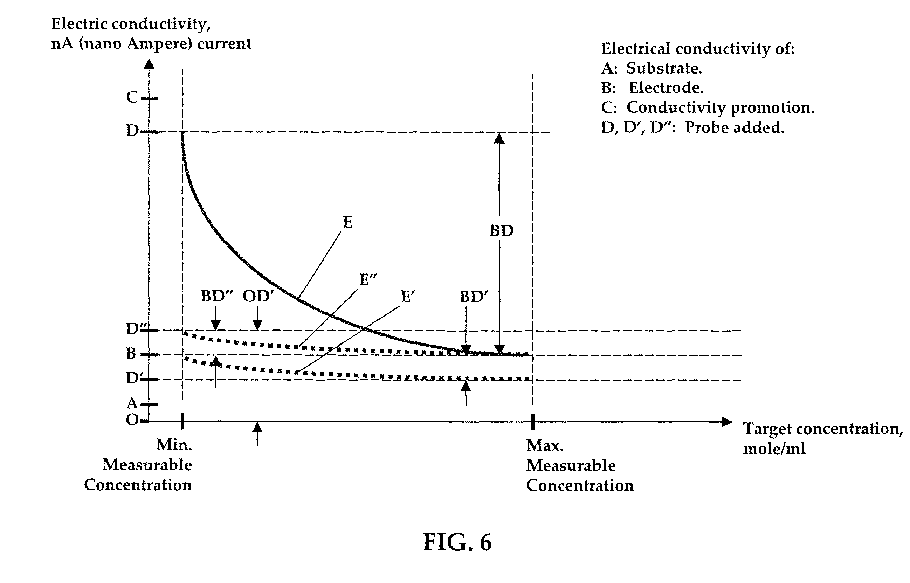

FIG. 6 schematically explains how the electrosensing chip and method of the present invention is practically useful. The graph depicts the relationship of the electrical conductivity of a test sample with respect to the target antigen concentration in the sample.

Nomenclature A, B, C, D, D' and D" in FIG. 6 along the vertical scale, the electrical conductivity, are, respectively, the electrical conductivity of the sensor chip system at various stages of its fabrication:
A: substrate
B: electrode
C: conductivity promotion
D, D', D": antibody probes added

Conventional electrosensing measures sample conductivity in terms of current in the small current reading range (BD' or BD", whether the addition of probes slightly decreases or increases overall conductivity respectively) for a wide range of sample concentrations. The current reading range is so small to be practically useful even to discern the presence of the target, less any possibility of making sense of the sample concentration curvature, E' or E", to any acceptable reading resolution.

By contrast, the use of conductivity promoting molecules, in a sense, amplifies the detection range of target (BD), allowing for determination of target concentration with good resolution and therefore accuracy. This is because, as clearly illustrated by the characteristic curve E in FIG. 6, target detection and measurement within the wide measurement correspondence range, a linear or non-linear relationship between the target concentration in the fluidic environment and the correspondingly measured current therein, makes interpretation of the instrumentation reading much more easier.

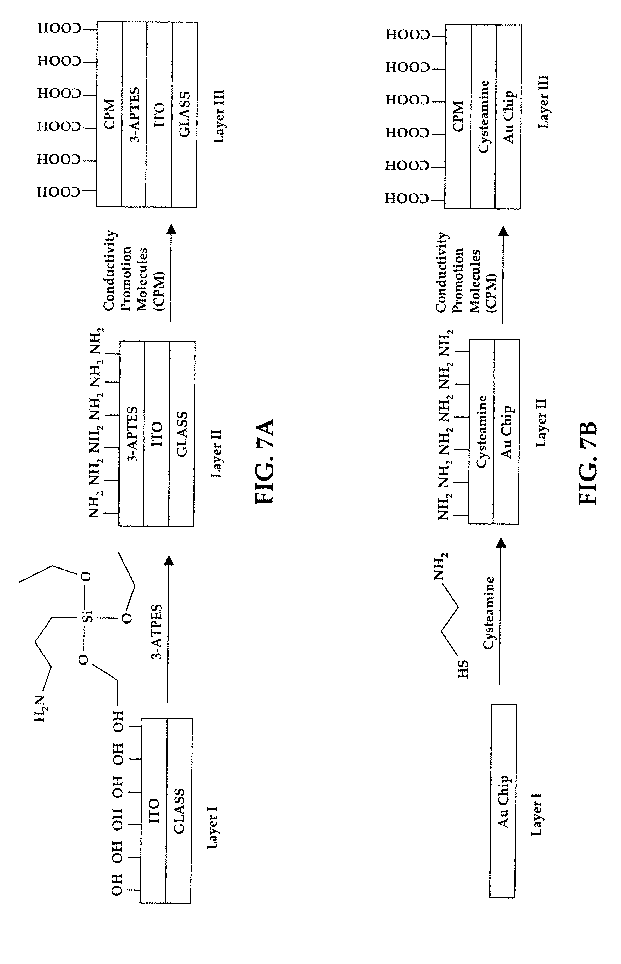

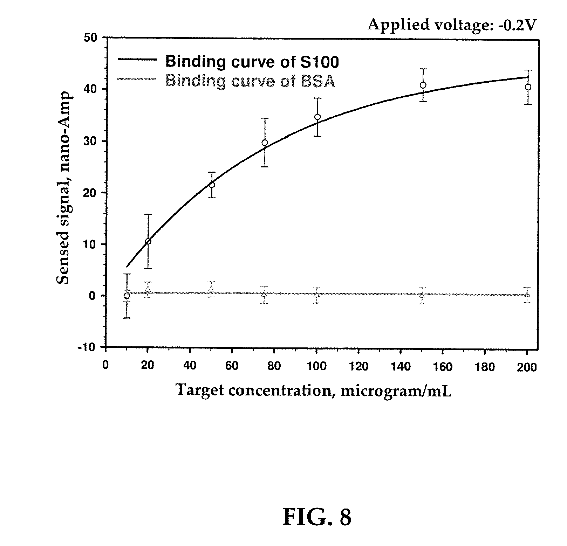

FIGS. 7A and 7B illustrate two examples of the process for surface modification of sensor chips of the present invention that promotes electrical conductivity in the test system. FIGS. 8-10 respectively show results of various sample testing using sensors of the present invention.

FIG. 8 shows the binding curves of S100 antigens in tests to anti-S100 monoclonal antibody using -0.2V measurement voltages. Sensor chips with anti-S100 monoclonal antibody (mAb) probe are used to detect antigen S100. Binding curve in FIG. 8 is obtained under a -0.2V test voltage, which yields a clearly discernable relationship mapping a sample concentration with a range from 0 to 200 micrograms per milli-liter onto a sensed current of 0 to 40 nano-ampere. BSA (bovine serum albumin) is used as a control sample in the test.

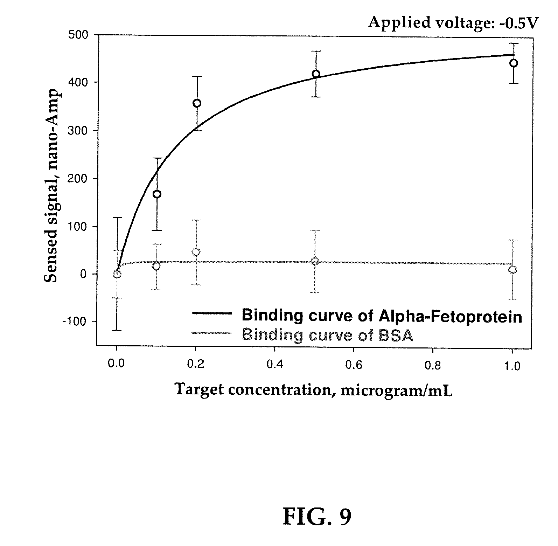

FIG. 9 shows the binding curve of alpha fetoprotein in tests to anti-alpha fetoprotein monoclonal antibody using -0.5V measurement voltage at different antigen concentrations. Sensor chips with anti-alpha fetoprotein monoclonal antibody (mAb) probe are used to detect antigen alpha fetoprotein. The binding curve is obtained under a -0.5V test voltage for test samples with concentrations ranging from 0 to 1 microgram per milliliter. The tests yield a clearly discernable relationship mapping a sample concentration onto a corresponding range of sensed current. BSA (bovine serum albumin) is used as a control sample in the test.

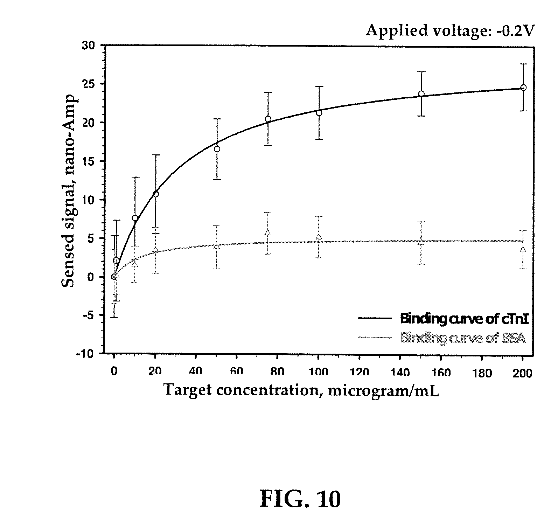

FIG. 10 shows the binding curve of anti-(tropolin I) monoclonal antibody to tropolin in a test using -0.2V measurement voltage. A sensor chip with anti-(tropolin I) monoclonal antibody (mAb) probe is used to detect antigen tropolin. The binding curve is obtained under a -0.2V test voltage for test samples with concentration ranging from 0 to 200 microgram per milliliter. The test yields a clearly discernable relationship mapping a sample concentration onto a corresponding range of sensed current. BSA (bovine serum albumin) is used as a control sample in the test.

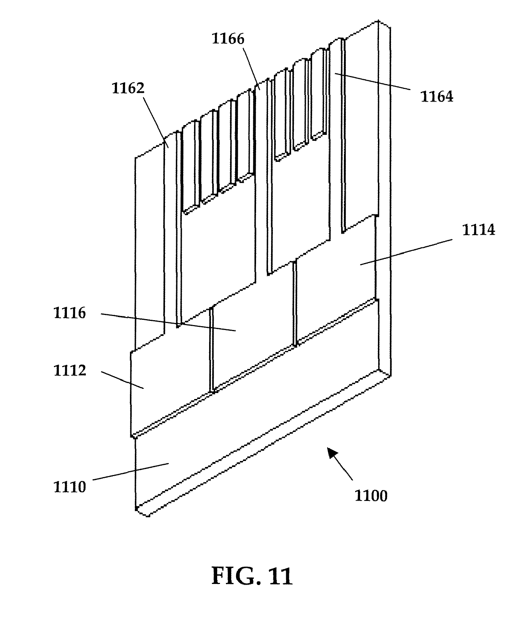

FIG. 11 illustrates the physical outline of an embodiment of the sensor chip of the present invention. In this implementation of a flat chip configuration, a sensor chip 1100 with a flat electrodes outline can have a glass substrate 1110 with plated electrodes 1112, 1114 and 1116 and edge connectors 1162, 1164 and 1166 similar to those found on printed circuit boards. Three electrodes are, in this example, with a center reference electrode 1116 between the two positive 1112 and negative 1114 functional electrodes. Some edge connectors are reserved for future functions. Typical metallic conductor plates for sensor function electrodes include Au, Ag, Cu, Ni, etc., or, flexible films of conductive material may also be used. Typical inert metal plate for the reference electrode includes Pt and others. Edge connectors that connect the chip to a processor can use those found in PCB. Typical thickness of these plating is about 2,000 angstrom.

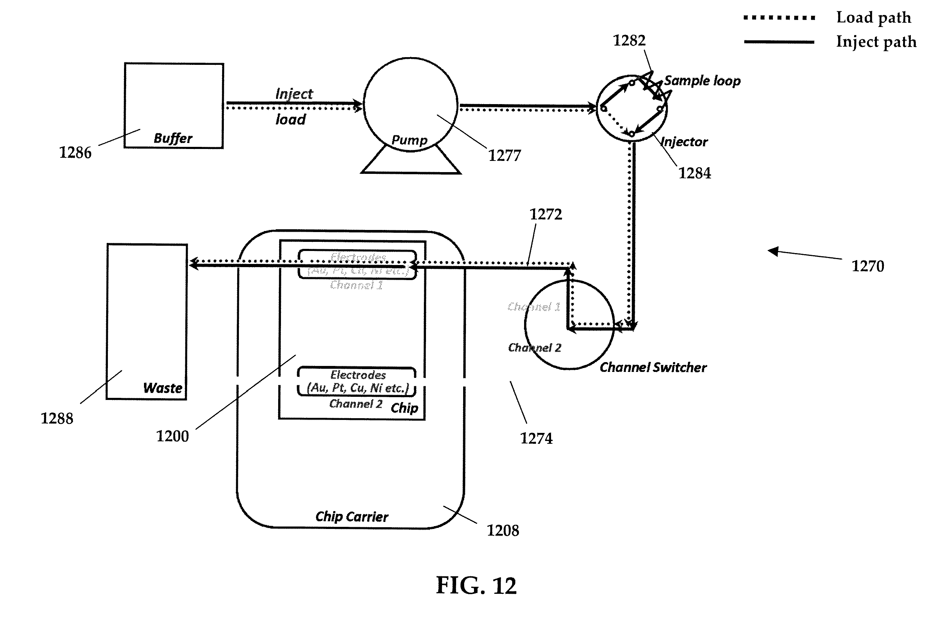

FIG. 12 outlines the flow channel configuration of a sensor chip processing machine 1270 in accordance with an embodiment of the present invention. Two fluid channels 1272 and 1274 are used in the processing machine implementation. When a sensor chip 1200, optionally carried in a chip carrier 1208, is used for the testing of designated target substance, the sample is loaded into the sample loop 1282 by the injector 1284 via the load path, shown in the drawing as dotted lines. Then, with a pump 1277, a buffer fluid pumps the sample to pass through the surface of the electrodes of the sensor chip using a buffer supplied by a reservoir 1286. The test and buffer fluid then exit to a waste tank 1288 for later disposal.

While the above is a full description of the specific embodiments, various modifications, alternative constructions and equivalents may be used. For example, for the crucial innovativeness of the present invention, the preferred embodiments described and shown in the drawing only illustrate one of several possible configurations of implementation of electrical conductivity promotion in the sensor chip system, namely, the use of conductivity promotion molecules also as a means of antibody's linkage to the electrodes illustrated in FIGS. 4's and 5's. It is, however, easily comprehensible for anyone skilled in the art that other arrangements of the use of the conductivity promotion molecules in the system are certainly possible. For example, slightly different arrangements including modifying the antibody with the conductivity promotion molecules, adding conductivity promotion molecules in the buffer fluid pumped through the sensor chip surface or used in the test sample, and any combination of any of these arrangements are equally applicable, as subsequent and additional experimental results have already confirmed. Therefore, the above description and illustrations should not be taken as limiting the scope of the present invention.

## Claims

1. A sensor (400) for electrosensing an antigen (432) in a test sample comprising
two electrodes (412, 414) electrically disconnected and physically separated from each other; and
a layer of antibody (422) immobilized on the surface of at least one of said electrodes (412, 414), said antibody (422) having specific binding reactivity with said antigen (432).

2. The sensor (400) of claim 1 wherein said antibody (422) capturing said antigen (432) present in said test sample mixed in a buffer solution that comes into contact with said antibody-populated electrodes (412, 414) thereby altering electrical conductivity characteristic across said two electrodes, whereby an amount representative of said altering providing an indication for electrosensing of said antigen (432).

3. The sensor (400) of claim 1 wherein said electrosensing is a measurement of current across said electrodes (412, 414) under either DC or an AC.

4. The sensor (400) of claim 1 wherein said electrosensing is a measurement of capacitance across said electrodes (412, 414).

5. The sensor (400) of claim 1 wherein said electrodes (412, 414) bare made of material selected from the group consisting of Au, Ag, Cu and Ni.

6. The sensor (400) of claim 1 wherein said electrodes (412, 414) are on a surface of a non-conductive flat substrate (410) of said sensor.

7. The sensor (500) of claim 1 wherein said electrodes (512, 514) are positioned oppositely facing each other on a non-conductive tubular substrate (510) of said sensor.

8. A sensor (400) for electrosensing an antigen (432) in a test sample comprising
two electrodes (412, 414) electrically disconnected and physically separated from each other;
a layer of antibody (422) immobilized on the surface of at least one of said electrodes (412, 414), said antibody (422) having specific binding reactivity with said antigen (432); and
conductivity promotion molecules (442) tethered over and/or distributed between said antibody-populated electrodes (412, 414) for improving electrical conductivity characteristics across said two electrodes.

9. The sensor (400) of claim 8 wherein said antibody (422) capturing said antigen (432) present in said test sample mixed in a buffer solution that comes into contact with said antibody-populated electrodes (412, 414) thereby altering said improved electrical conductivity characteristic across said two electrodes, whereby an amount representative of said altering providing an indication for electrosensing of said antigen (432).

10. A method of electrosensing an antigen (432) in a test sample using a sensor (400), said sensor (400) having two electrodes (412, 414) electrically disconnected and physically separated from each other and a layer of antibody (422) immobilized on the surface of at least one of said electrodes (412, 414), said antibody (422) having specific binding reactivity with said antigen (432); said method comprising
measuring electrically across said electrodes (412, 414) after said test sample comes into contact with said antibody-populated electrodes.

11. The method of claim 10 wherein said antibody (422) capturing said antigen (432) present in said test sample thereby altering electrical conductivity characteristic across said two electrodes (412, 414), whereby an amount representative of said altering providing an indication for electrosensing of said antigen (432).

12. The method of claim 10 wherein said electrosensing is a measurement of current across said electrodes (412, 414) under either DC or an AC.

13. The method of claim 10 wherein said electrosensing is a measurement of capacitance across said electrodes (412, 414).

14. The method of claim 10 wherein said electrodes (412, 414) are made of material selected from the group consisting of Au, Ag, Cu and Ni.

15. The method of claim 10 wherein said electrodes (412, 414) are on a surface of a non-conductive flat substrate (410) of said sensor.

16. The method of claim 10 wherein said electrodes (512, 514) are positioned oppositely facing each other on a non-conductive tubular substrate (510) of said sensor (500).

17. A method of electrosensing an antigen (432) in a test sample using a sensor (400), said sensor (400) having two electrodes (412, 414) electrically disconnected and physically separated from each other and a layer of antibody (422) immobilized on the surface of at least one of said electrodes (412, 414), said antibody (422) having specific binding reactivity with said antigen (432); said method comprising
tethering conductivity promotion molecules (442) over and/or distributing between said antibody-populated electrodes (412, 414) for improving electrical conductivity characteristics across said two electrodes; and
measuring electrically across said electrodes (412, 414) after said test sample comes into contact with said antibody-populated electrodes.

18. The method of claim 17 wherein said antibody (422) capturing said antigen (432) present in said test sample thereby altering said improved electrical conductivity characteristic across said two electrodes (412, 414), whereby an amount representative of said altering providing an indication for electrosensing of said antigen (432).
